(19) Europäisches Patentamt / European Patent Office / Office européen des brevets

(11) EP 4 674 832 A1

(12) **EUROPEAN PATENT APPLICATION**
published in accordance with Art. 153(4) EPC

(43) Date of publication:
07.01.2026 Bulletin 2026/02

(21) Application number: 24763492.6

(22) Date of filing: 01.02.2024

(51) International Patent Classification (IPC):
*C07C 17/389* (2006.01)   *C07C 21/18* (2006.01)

(52) Cooperative Patent Classification (CPC):
C07C 17/389; C07C 21/18

(86) International application number:
PCT/JP2024/003331

(87) International publication number:
WO 2024/181008 (06.09.2024 Gazette 2024/36)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 28.02.2023 JP 2023030192

(71) Applicant: AGC INC.
Chiyoda-ku,
Tokyo 1008405 (JP)

(72) Inventors:
• OTSUKA, Tetsuo
Tokyo 100-8405 (JP)
• MATSUNAMI, Asuka
Tokyo 100-8405 (JP)
• MITAKE, Akihito
Tokyo 100-8405 (JP)
• SHIOTA, Hidefumi
Tokyo 100-8405 (JP)
• KAMATSUKA, Tatsuya
Tokyo 100-8405 (JP)

(74) Representative: Müller-Boré & Partner
Patentanwälte PartG mbB
Friedenheimer Brücke 21
80639 München (DE)

(54) **METHOD FOR PURIFYING VINYLIDENE FLUORIDE**

(57) A method of purifying vinylidene fluoride is provided. The method comprising: contacting a first fluid including vinylidene fluoride as a main component and further including trifluoromethane with a synthetic zeolite 4A, obtaining a second fluid in which the mass ratio of the content of vinylidene fluoride to the total content of the vinylidene fluoride and trifluoromethane is higher than the mass ratio of the content of the vinylidene fluoride to the total content of the vinylidene fluoride and the trifluoromethane in the first fluid.

EP 4 674 832 A1

## Description

Technical Field

The present disclosure relates to a method of purifying vinylidene fluoride.

Background Art

Vinylidene fluoride is useful as a monomer for a fluorine resin.

For example, Patent Literature 1 describes a method of producing 2,3,3,3-tetrafluoropropene, wherein the method includes: (a) a step of supplying a mixture, obtained by mixing methyl chloride at a molar ratio of from 3.2 to 4.7 with respect to chlorodifluoromethane, into a reaction container; (b) a step of supplying a heat medium to the mixture in the step (a) to bring the heat medium into contact with the mixture, to generate a second mixture including 2,3,3,3-tetrafluoropropene and methyl chloride; (c) a step of drying the second mixture in the step (b), to obtain an anhydrous second mixture; (d) a step of bringing the anhydrous second mixture in the step (c) into contact with a molecular sieve having a pore diameter of 4 Å, to obtain a third mixture containing no methyl chloride; and (e) a step of separating 2,3,3,3-tetrafluoropropene from the third mixture in the step (d).

Citation List

Patent Literature

Patent Literature 1: Japanese Patent No. 7014709

## SUMMARY OF INVENTION

Technical Problem

However, an objective of Patent Literature 1 is to separate and obtain 2,3,3,3-tetrafluoropropene, and vinylidene fluoride (VdF) is regarded as an impurity in Patent Literature 1. In contrast, a purification method of which an objective is to separate and obtain VdF from a composition including VdF as a main component has been demanded.

One embodiment of the present invention addresses a task of providing a method of purifying VdF, by which high-purity VdF can be obtained from a composition including VdF as a main component.

Solution to Problem

The present disclosure includes the following aspects.

<1> A method of purifying VdF, the method comprising: contacting a first fluid including VdF as a main component and further including trifluoromethane with a synthetic zeolite 4A, and obtaining a second fluid in which a mass ratio of a content of VdF to a total content of the VdF and trifluoromethane is higher than a mass ratio of a content of the VdF to a total content of the VdF and the trifluoromethane in the first fluid.
<2> The method of purifying VdF according to <1>, wherein a ratio of a flow amount of the first fluid to an amount of the synthetic zeolite 4A filled into a reaction vessel is 0.160 [(g/h)/g] or less.
<3> The method of purifying VdF according to <1> or <2>, wherein the first fluid is a gas.
<4> The method of purifying VdF according to any one of <1> to <3>, wherein the content of the VdF in the second fluid is 99% by mass or more with respect to a total amount of the second fluid.

Advantageous Effects of Invention

In accordance with one embodiment of the invention, a method of purifying VdF, by which high-purity VdF can be obtained from a composition including VdF as a main component, is provided.

## DESCRIPTION OF EMBODIMENTS

In the present disclosure, a numerical range expressed by "x to y" means a range including the values of x and y as the minimum and maximum values, respectively.

In a numerical range expressed in a stepwise manner in the present disclosure, the upper or lower limit value expressed in a certain numerical range may be replaced by the upper or lower limit value in another numerical range expressed in a stepwise manner. In a numerical range expressed in the present disclosure, the upper or lower limit value expressed in a certain numerical range may be replaced by values described in Examples.

In the present disclosure, a combination of two or more preferred aspects is a more preferred aspect.

In the present disclosure, in a case in which plural kinds of substances corresponding to each component exist, the amount of each component means, unless otherwise specified, the total amount of the plural kinds of substances.

[Method of Purifying VdF]

In a method of purifying VdF of the present disclosure, a first fluid including VdF as a main component and further including trifluoromethane (R23) is brought into contact with a synthetic zeolite 4A, to obtain a second fluid in which the mass ratio of the content of VdF to the total content of VdF and R23 is higher than the mass ratio of the content of VdF to the total content of VdF and R23 in the first fluid.

In accordance with the method of purifying VdF of the present disclosure, a component except VdF can be efficiently removed from a composition including VdF as a main component, to obtain high-purity VdF.

VdF has a boiling point of -83°C, and R23 has a boiling point of -82°C. VdF and R23 have the similar boiling points, and therefore, it is difficult to separate and purify VdF and R23 by distillation. VdF is useful as a monomer for a fluorine resin. However, for example, in a case in which a polymerization reaction is performed using a composition including VdF and R23 as an impurity, R23 that is not consumed in the polymerization reaction accumulates in the interior of a reaction vessel, whereby the polymerization reaction is inhibited. As a result, the rate of the polymerization reaction is decreased, or the polymerization reaction is stopped, whereby a polymer of interest is prone to be prevented from being obtained. It is desirable to produce high-purity VdF.

The present inventors found that a first fluid including VdF as a main component and further including R23 is brought into contact with a synthetic zeolite 4A, whereby R23 can be efficiently removed to increase the mass ratio of the content of VdF to the total content of VdF and R23.

An objective of the production method described in Patent Literature 1 is to produce HFO-1234yf, and VdF is regarded as an impurity. VdF is regarded as an impurity, and therefore, a purification method using a composition including VdF as a main component is not described. It is not efficient to remove a component except VdF from a composition that does not include VdF as a main component to obtain high-purity VdF.

Specifically, about 4% of R23 and about 10% of VdF are included in 355 g/h (total of the supply amount of chlorodifluoromethane and methyl chloride which are source gases) of crude gas, and adsorbed using 2.4 kg of a molecular sieve of 4 Å in the production method described in Patent Literature 1. At 79 minutes after the start, the content of VdF is 7.22%, the content of R23 is 15.34%, and the mass ratio of the content of VdF to the total content of VdF and R23 is increased. This is considered to be because a breakthrough occurs in a molecular sieve of 4 Å, and an equilibrium adsorption amount is reached. The amount of adsorbed R23 until 79 minutes is about 19 g, corresponding to 0.0079 g per gram of molecular sieve of 4 Å. The amount of adsorbed R23 is very small, and the purification of VdF is not efficient.

<First Fluid>

The first fluid used in the method of purifying VdF of the present disclosure includes VdF as a main component, and includes R23.

In the present disclosure, "main component" means that the amounts of components other than the component are relatively smaller. In other words, "main component" means that the content of the component is the highest in a composition including the component.

In other words, the content of VdF is the highest in the first fluid including VdF as a main component.

The content of VdF in the first fluid is preferably 50% by mass or more, more preferably 60% by mass or more, still more preferably 70% by mass or more, and particularly preferably 80% by mass or more with respect to the total amount of the first fluid from the viewpoint of more efficiently purifying VdF. The upper limit value of the content of VdF is not particularly limited, and is, for example, less than 99.5% by mass.

R23 may be inhibited from being adsorbed in the synthetic zeolite 4A, and R23 may be desorbed from the synthetic zeolite 4A in a case in which VdF is not a main component, and the first fluid includes a large amount of component except VdF. In contrast, the adsorption of R23 in the synthetic zeolite 4A may immediately proceed, and the mass ratio of the content of VdF to the total content of VdF and R23 may be increased in a case in which VdF is a main component in the first fluid. High-purity VdF can be efficiently obtained by allowing VdF to be the main component even in the case of using a small amount of synthetic zeolite 4A.

The content of R23 in the first fluid is more than 0% by mass. A case in which R23 is not a main component is acceptable, and the content of R23 is not particularly limited.

The content of R23 in the first fluid is preferably less than 50% by mass, more preferably 40% by mass or less, still more preferably 30% by mass or less, and particularly preferably 20% by mass or less with respect to the total amount of the first fluid.

The first fluid may include another component except VdF and R23, and the smaller content of the other component is preferred from the viewpoint of efficiently obtaining high-purity VdF. Examples of such another component include fluoroolefins except VdF, and hydrofluorocarbons except R23.

The content of the other component in the first fluid is preferably 20% by mass or less, and more preferably 10% by mass or less with respect to the total amount of the first fluid.

The first fluid may be gas, or may be liquid. The first fluid is preferably gas from the viewpoint of eliminating the need of a production facility or the like for liquefying a composition. In contrast, the first fluid is preferably liquid from the viewpoint of the amount of adsorbed R23.

As the first fluid, for example, a reaction product containing VdF, obtained by allowing various raw materials to react with each other, can be used for the purpose of producing VdF.

For example, the reaction product containing VdF can be obtained by thermal decomposition reaction using chlorodifluoroethane as a raw material. The reaction product containing VdF can be obtained by dehydrochlorination reaction using chlorodifluoromethane and chloromethane as raw materials.

<Synthetic Zeolite 4A>

In the method of purifying VdF of the present disclosure, the first fluid is brought into contact with the synthetic zeolite 4A.

For example, a synthetic zeolite 3A has a small pore diameter, and therefore, it is impossible to allow the synthetic zeolite 3A to adsorb R23. A synthetic zeolite 5A has a large pore diameter, and therefore, VdF is also adsorbed together with R23 in the synthetic zeolite 5A. Accordingly, it is difficult to obtain high-purity VdF in the case of using the synthetic zeolite 3A or the synthetic zeolite 5A. In contrast, VdF is inhibited from being adsorbed in the synthetic zeolite 4A, and R23 is adsorbed in the synthetic zeolite 4A. Therefore, high-purity VdF can be obtained by the method of purifying VdF of the present disclosure.

The synthetic zeolite 4A is a synthetic zeolite that has a type A crystal structure and is represented by the following Formula 1.

$$(Na_aM^1_bM^2_c)(Al_mSi_nO_{2(m+n)}) \cdot xH_2O \qquad \text{Formula 1}$$

In Formula 1,

$M^1$ is at least one selected from the group consisting of Li and K,
$M^2$ is at least one selected from the group consisting of Ca, Mg, and Ba,
each of a, b, c, x, m, and n is independently an integer 1 or more, and
a, b, c, m, and n satisfy the following Formulas 2, 3, and 4.

$$a + b + 0.5\,c = m \qquad \text{Formula 2}$$

$$a\,(a + b + 0.5\,c) \geq 0.6 \qquad \text{Formula 3}$$

$$n/m \geq 1.0 \qquad \text{Formula 4}$$

It is preferable that a, b, c, m, and n satisfy the following Formulas 3a and 4a.

$$a\,(a + b + 0.5\,c) \geq 0.9 \qquad \text{Formula 3a}$$

$$1.3 > n/m \geq 1.0 \qquad \text{Formula 4a}$$

The synthetic zeolite 4A is preferably a synthetic zeolite represented by the following Formula 5.

$$Na_{12}[(AlO_2)_{12}SiO_2)_{12}] \cdot xH_2O \qquad \text{Formula 5}$$

Whether or not to have the type A crystal structure can be confirmed using an X-ray diffraction method.

Examples of the synthetic zeolite 4A include type A synthetic zeolites denoted as 4A. Examples of commercially available products thereof include MOLECULAR SIEVE 4A (manufactured by UNION SHOWA K.K.).

Activation treatment of the synthetic zeolite 4A may be performed before use of the synthetic zeolite 4A in the purification of VdF. Examples of a method of the activation treatment include a method of performing heat treatment by dry gas at 100 to 400°C and a method of performing heat treatment under reduced pressure. In the case of performing the activation treatment, the synthetic zeolite 4A is activated, to improve the efficiency of removing R23.

A form of use of the synthetic zeolite 4A is not particularly limited. The first fluid may be allowed to flow through an apparatus into which the synthetic zeolite 4A is filled. Alternatively, the first fluid may be filled into a container into which the synthetic zeolite 4A is filled, and the second fluid may be extracted after a lapse of predetermined time.

<Second Fluid>

In the method of purifying VdF of the present disclosure, the second fluid is obtained by bringing the first fluid into contact with the synthetic zeolite 4A. The mass ratio of the content of VdF to the total content of VdF and R23 (that is, "VdF/(VdF + R23)") in the second fluid is higher than that in the first fluid.

The content of VdF in the second fluid is preferably 90% by mass or more, more preferably 95% by mass or more, still more preferably 99% by mass or more, and particularly preferably 99.5% by mass or more with respect to the total amount of the second fluid.

The content of R23 in the second fluid is preferably 1% by mass or less, and more preferably 0.5% by mass or less with respect to the total amount of the second fluid.

<Method of Bringing First Fluid into Contact with Synthetic Zeolite 4A>

As described above, the first fluid may be gas, or may be liquid. A contact method in a gas phase, and a contact method in a liquid phase are described below.

-Contact Method in Gas Phase-

A method in which an adsorption layer into which the synthetic zeolite 4A is filled is formed, and the first fluid is allowed to flow through the adsorption layer is preferred as the method in which the first fluid is gas, and the first fluid is brought into contact with the synthetic zeolite 4A in a gas phase. The method in which the first fluid is allowed to flow through the adsorption layer may be batch-type, or may be continuous-type. The method of purifying VdF of the present disclosure is preferably performed in gas-phase continuous-flow type using a fixed bed reaction vessel from the viewpoint of production efficiency.

Examples of the reaction vessel used in the case of allowing the first fluid to flow through the adsorption layer include a known reaction vessel into which the synthetic zeolite 4A can be filled to form an adsorption layer. Examples of a material for the reaction vessel include glass, iron, nickel, or alloys including glass, iron, and nickel as main components, and fluorine resins such as tetrafluoroethylene-perfluoro(alkyl vinyl ether) copolymers (PFAs).

The number of such adsorption layers may be one, or two or more. In a case in which the adsorption layers are two or more, the adsorption layers may be parallel, or may be in series.

In the case of the contact with the synthetic zeolite 4A, the temperature of the first fluid is not particularly limited, and is preferably 120°C or less, more preferably 100°C or less, and still more preferably 80°C or less from the viewpoint of obtaining VdF with higher purity. The temperature of the first fluid is preferably -30°C or more from the viewpoint of the adsorption efficiency of the synthetic zeolite 4A.

In the case of the contact with the synthetic zeolite 4A, the pressure (gauge pressure) of the first fluid is not particularly limited, and is preferably from 0 to 5000 kPa, and more preferably from 0 to 3000 kPa from the viewpoint of obtaining VdF with higher purity.

Contact time for which the first fluid and the synthetic zeolite 4A are brought into contact with each other is not particularly limited, and is, for example, from 1 to 60 seconds from the viewpoint of obtaining VdF with higher purity.

The ratio of the flow rate [(g/h)] of the first fluid to the amount [(g)] of synthetic zeolite 4A filled in the reaction vessel is preferably 0.200 [(g/h)/g] or less, more preferably 0.160 [(g/h)/g] or less, and still ore preferably 0.150 [(g/h)/g] or less. In a case in which the above-described ratio is 0.200 [(g/h)/g] or less, time to contact with the synthetic zeolite 4A becomes longer, and VdF with higher purity is obtained.

In addition, "g/hour" which is the unit of the flow rate of the first fluid means the mass per hour.

-Contact Method in Liquid Phase-

A method in which an adsorption layer into which the synthetic zeolite 4A is filled is formed, and the first fluid is allowed to flow through the adsorption layer is preferred as the method in which the first fluid is liquid, and the first fluid is brought into contact with the synthetic zeolite 4A in a liquid phase. A method in which the synthetic zeolite 4A and the first fluid are mixed, and stirred, if necessary, in a reaction vessel into which the synthetic zeolite 4A is filled is also acceptable. In a method in which the synthetic zeolite 4A and the first fluid are mixed in the reaction vessel, the synthetic zeolite 4A and the second fluid obtained by purification can be separated from each other by precipitation or filtration after the purification. These methods may be batch-type, or may be continuous-type.

Examples of the reaction vessel used in the case of allowing the first fluid to flow through the adsorption layer include a known reaction vessel into which the synthetic zeolite 4A can be filled to form an adsorption layer. Examples of a material for the reaction vessel include glass, iron, nickel, or alloys including glass, iron, and nickel as main components, and fluorine resins such as tetrafluoroethylene-perfluoro(alkyl vinyl ether) copolymers (PFAs).

Examples of the reaction vessel used in the case of mixing the first fluid with the synthetic zeolite 4A include autoclaves.

In the case of the contact with the synthetic zeolite 4A, the temperature of the first fluid is not particularly limited, and is preferably 50°C or less, more preferably 35°C or less, and still more preferably 10°C or less from the viewpoint of obtaining VdF with higher purity. The temperature of the first fluid is preferably -30°C or more from the viewpoint of the adsorption efficiency of the synthetic zeolite 4A.

In the case of the contact with the synthetic zeolite 4A, the pressure (gauge pressure) of the first fluid is not particularly limited, and is preferably from 400 to 5000 kPa, and more preferably from 400 to 3000 kPa from the viewpoint of obtaining VdF with higher purity. Examples

The present disclosure is specifically described in detail below with reference to Examples. However, the present disclosure is not limited to these Examples.

[Examples 11 to 14]

Examples 12 to 14 are examples, and Example 11 is a comparative example.

A synthetic zeolite 4A (product name "MOLECULAR SIEVE 4A", manufactured by KANTO CHEMICAL CO., INC.) and a first fluid including VdF and R23 were put in a 75-mL cylinder. In such a case, the temperature of the first fluid was 25°C. In Examples 11 to 14, the amount of loaded synthetic zeolite 4A and the contents of VdF and R23 in the first fluid ("Content before contact" in Table 1) were adjusted as set forth in Table 1. After a lapse of 24 hours, a gas phase and a liquid phase were sampled, and compositions ("Content after contact" in Table 1) were measured using gas chromatography. Based on the measurement results, the equilibrium adsorption amount of R23 adsorbed in the synthetic zeolite 4A was calculated.

[Table 1]

| | Synthetic zeolite 4A | Content before contact | | Content after contact [% by mass] | | Equilibrium adsorption amount of |
|---|---|---|---|---|---|---|
| | [g] | [% by mass] | | | | R23 [g/g] |
| | | VdF | R23 | VdF | R23 | |
| Example 11 | 42 | 8.3 | 91.7 | 8.7 | 91.3 | 0.186 |
| Example 12 | 30 | 89.2 | 10.8 | 91.1 | 8.9 | 0.137 |
| Example 13 | 31 | 96.0 | 4.0 | 97.1 | 2.9 | 0.071 |
| Example 14 | 30 | 99.4 | 0.6 | 99.7 | 0.3 | 0.021 |

Based on Table 1, it was found that the contact of the first fluid including VdF as a main component and further including R23 with the synthetic zeolite 4A allows the equilibrium adsorption amount of R23 to be much higher than in a conventional case, and high-purity VdF to be efficiently obtained.

[Examples 21 to 23]

Example 21 is Example, and Examples 22 and 23 are Comparative Examples.

In Example 21, a first fluid including VdF and R23 at 40°C was supplied to 52.5 g of synthetic zeolite 4A (product name "MOLECULAR SIEVE 4A", manufactured by JUNSEI CHEMICAL CO., LTD.) at a flow rate of 25 mL/min (4.3 g/h). In the first fluid, the content of R23 was set at 1083 ppm. After a lapse of predetermined, the composition of outlet gas, and the gas flow rate of the outlet gas were measured.

In Example 22, a first fluid was allowed to flow, and the composition of outlet gas, and the gas flow rate of the outlet gas were measured after a lapse of predetermined time, by a method similar to the method in Example 21 except that a synthetic zeolite 3A (product name "MOLECULAR SIEVE 3A", manufactured by JUNSEI CHEMICAL CO., LTD.) was used instead of the synthetic zeolite 4A.

In Example 23, a first fluid was allowed to flow, and the composition of outlet gas, and the gas flow rate of the outlet gas were measured after a lapse of predetermined time, by a method similar to the method in Example 21 except that a synthetic zeolite 5A (product name "MOLECULAR SIEVE 5A", manufactured by JUNSEI CHEMICAL CO., LTD.) was used instead of the synthetic zeolite 4A.

[Table 2]

| | Example 21 | | Example 22 | | Example 23 | |
|---|---|---|---|---|---|---|
| Time [min] | Content of R23 [ppm] | Gas flow rate of outlet gas [mL/min] | Content of R23 [ppm] | Gas flow rate of outlet gas [mL/min] | Content of R23 [ppm] | Gas flow rate of outlet gas [mL/min] |
| 60 | 210 | 25 | 975 | 25 | 0 | 0 |
| 120 | 266 | 25 | 960 | 25 | 0 | 0 |
| 180 | 285 | 25 | 962 | 25 | 4 | 0 |
| 240 | 292 | 25 | 1062 | 25 | 329 | 8 |
| 300 | 318 | 25 | - | - | 811 | 19 |
| 360 | 372 | 25 | - | - | 980 | 23 |

As set forth in Table 2, it was found that high-purity VdF was efficiently obtained in the case of bringing the first fluid including VdF as a main component and further including R23 into contact with the synthetic zeolite 4A in Example 21.

In Example 22, no high-purity VdF was obtained even in the case of bringing the first fluid into contact with the synthetic zeolite 3A. This is considered to be because the synthetic zeolite 3A has the low ability of adsorbing R23.

In Example 23, no high-purity VdF was obtained even in the case of bringing the first fluid into contact with the synthetic zeolite 5A. This is considered to be because the gas flow rate of the outlet gas was 0 mL/min for predetermined time after the contact, and VdF was adsorbed together with R23 in the synthetic zeolite 5A.

[Example 31]

In Example 31, a first fluid was allowed to flow by a method similar to the method in Example 21 except that the flow rate of the first fluid was set at 50 mL/min (8.6 g/h). After a lapse of predetermined time, the composition of outlet gas was measured. The ratio of the flow rate of the first fluid to the amount of filled synthetic zeolite 4A was about 0.082 [(g/h)/g] in Example 21, or about 0.164 [(g/h)/g] in Example 31.

[Table 3]

| | Example 21 | Example 31 |
|---|---|---|
| Time [min] | Content of R23 [ppm] | Content of R23 [ppm] |
| 60 | 210 | 415 |
| 120 | 266 | 496 |
| 180 | 285 | 544 |
| 240 | 292 | 610 |
| 300 | 318 | 617 |
| 360 | 372 | 651 |

Based on Table 3, it was found that high-purity VdF was further efficiently obtained in a case in which the ratio of the flow rate of the first fluid to the amount of synthetic zeolite 4A filled in the reaction vessel was 0.160 [(g/h)/g] or less.

The entire content of the disclosure by Japanese Patent Application No. 2023-030192 filed on February 28, 2023 is incorporated herein by reference. All documents, patent applications, and technical standards described in this specification are herein incorporated by reference to the same extent as if each individual document, patent application, or technical standard was specifically and individually indicated to be incorporated by reference.

**Claims**

1.  A method of purifying vinylidene fluoride, the method comprising:

    contacting a first fluid, comprising vinylidene fluoride as a main component and further comprising trifluoromethane, with a synthetic zeolite 4A; and
    obtaining a second fluid in which a mass ratio of a content of vinylidene fluoride to a total content of the vinylidene fluoride and trifluoromethane is higher than a mass ratio of a content of the vinylidene fluoride to a total content of the vinylidene fluoride and the trifluoromethane in the first fluid.

2.  The method of purifying vinylidene fluoride according to claim 1, wherein a ratio of a flow amount of the first fluid to an amount of the synthetic zeolite 4A filled into a reaction vessel is 0.160 (g/h)/g or less.

3.  The method of purifying vinylidene fluoride according to claim 1 or 2, wherein the first fluid is a gas.

4.  The method of purifying vinylidene fluoride according to claim 1 or 2, wherein the content of the vinylidene fluoride in the second fluid is 99% by mass or more with respect to a total amount of the second fluid.

## INTERNATIONAL SEARCH REPORT

| International application No. |
| --- |
| **PCT/JP2024/003331** |

| A. | CLASSIFICATION OF SUBJECT MATTER |
| --- | --- |

*C07C 17/389*(2006.01)i; *C07C 21/18*(2006.01)i
FI:  C07C17/389; C07C21/18

According to International Patent Classification (IPC) or to both national classification and IPC

| B. | FIELDS SEARCHED |
| --- | --- |

Minimum documentation searched (classification system followed by classification symbols)

C07C17/389; C07C21/18

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | WO 2015/072305 A1 (ASAHI GLASS COMPANY, LIMITED) 21 May 2015 (2015-05-21) claim 1, example 9, paragraph [0004] | 1-4 |
| Y | WO 2013/151070 A1 (ASAHI GLASS COMPANY, LIMITED) 10 October 2013 (2013-10-10) claim 1, table 1, paragraphs [0027], [0031], [0062] | 1-4 |
| A | JP 2009-543793 A (HONEYWELL INTERNATIONAL, INC.) 10 December 2009 (2009-12-10) | 1-4 |
| A | WO 2021/049605 A1 (KANTO DENKA KOGYO CO., LTD.) 18 March 2021 (2021-03-18) | 1-4 |
| A | CN 101704709 A (SHANDONG DONGYUE SHENZHOU NEW MATERIAL CO., LTD.) 12 May 2010 (2010-05-12) | 1-4 |

☐ Further documents are listed in the continuation of Box C.    ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **04 April 2024** | **16 April 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT
**Information on patent family members**

International application No.

**PCT/JP2024/003331**

| Patent document cited in search report | | | Publication date (day/month/year) | Patent family member(s) | | | Publication date (day/month/year) |
|---|---|---|---|---|---|---|---|
| WO | 2015/072305 | A1 | 21 May 2015 | (Family: none) | | | |
| WO | 2013/151070 | A1 | 10 October 2013 | (Family: none) | | | |
| JP | 2009-543793 | A | 10 December 2009 | US | 2008/0011159 | A1 | |
| | | | | WO | 2008/008695 | A1 | |
| | | | | EP | 2038043 | A1 | |
| WO | 2021/049605 | A1 | 18 March 2021 | US | 2022/0281785 | A1 | |
| | | | | CN | 114375287 | A | |
| | | | | KR | 10-2022-0061994 | A | |
| CN | 101704709 | A | 12 May 2010 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7014709 B

- JP 2023030192 A